Europäisches Patentamt

European Patent Office

Office européen des brevets

(11) Publication number: **0 277 211 B1**

(12) EUROPEAN PATENT SPECIFICATION

(45) Date of publication of patent specification: **01.06.94** (51) Int. Cl.5: **A61K 7/42, A61K 31/74**

(21) Application number: **87905543.2**

(22) Date of filing: **14.08.87**

(86) International application number:
**PCT/US87/02013**

(87) International publication number:
**WO 88/01164 (25.02.88 88/05)**

The file contains technical information submitted
after the application was filed and not included in
this specification

(54) POLYMERIC CARRIER COMPOSITIONS AND METHODS FOR THEIR PREPARATION AND USE.

(30) Priority: **15.08.86 US 896956**
**20.11.86 US 932613**
**13.01.87 US 2973**

(43) Date of publication of application:
**10.08.88 Bulletin 88/32**

(45) Publication of the grant of the patent:
**01.06.94 Bulletin 94/22**

(84) Designated Contracting States:
**AT BE CH DE FR GB IT LI LU NL SE**

(56) References cited:
**EP-A- 0 143 608      US-A- 4 154 917**
**US-A- 4 435 524      US-A- 4 477 467**
**US-A- 4 525 340      US-A- 4 542 069**
**US-A- 4 590 068**

(73) Proprietor: **Advanced Polymer Systems, Inc.**
**3696C Haven Avenue**
**Redwood City, CA 94063(US)**

(72) Inventor: **WON, Richard**
**3787 Nathan Way**
**Palo Alto, CA 94303(US)**
Inventor: **KATZ, Martin**
**5 Whitney Court**
**Menlo Park, CA 94025(US)**
Inventor: **CHENG, Chung, H.**
**1162 Robalo Court**
**San Jose, CA 95131(US)**

(74) Representative: **Stuart, Ian Alexander et al**
**MEWBURN ELLIS**
**York House**
**23 Kingsway**
**London WC2B 6HP (GB)**

Note: Within nine months from the publication of the mention of the grant of the European patent, any person
may give notice to the European Patent Office of opposition to the European patent granted. Notice of opposition
shall be filed in a written reasoned statement. It shall not be deemed to have been filed until the opposition fee
has been paid (Art. 99(1) European patent convention).

## Description

The present invention relates generally to the preparation of compositions and systems for the topical delivery of active substances to the skin. More particularly, the invention relates to the preparation of a rigid polymer bead delivery system for active substances such as infrared absorbants, insect repellants, steroids, emollients, and the like.

It is frequently desirable to topically deliver active ingredients to the human skin. In many cases, the active ingredients can be applied directly to the skin, either in a substantially pure form or in combination with a liquid vehicle. Such direct application, however, is limited in a number of respects. First, direct application allows rapid evaporation of volatile active substances, such as those listed above. Second, application of the active substances in substantially pure form can often cause toxic and/or allergic reactions, particularly in the case of infrared absorbants, insect repellants and steroids. While such adverse reactions can often be minimized by dilution of the active substance in a suitable liquid carrier, the consequent decrease in concentration frequently limits the effectiveness of the resulting combination for the intended purpose. Finally, many topically applied active substances have undesirable characteristics, such as an oily feel or a strong odor. While dilution of the pure active substance in a suitable liquid carrier can minimize such aesthetic objections, the resulting dilution will also reduce the effectiveness of the final product.

For these reasons, it would be desirable to provide delivery compositions or systems capable of providing controlled and prolonged delivery of active substances after they have been applied to the skin. Desirably, such delivery systems should also control any odor or toxicity which may be associated with the active substance, and should be suitable both for direct application to the skin and for combination in conventional liquid carriers.

Polymeric beads have been proposed for incorporating various active substances. European Patent No. 61,701 describes the preparation of relatively non-rigid polymeric beads for incorporating active substances, exemplified by emollients. Although such polymer delivery system will likely result in prolonged release of an active substance, the non-rigid beads allow the internal pore network incorporating the active substance to collapse as the substance is released, usually resulting in the entrapment and waste of residual active substance. Also, the European patent teaches a preparation procedure which requires the presence of the active substance during the polymerization of the bead material. Such a preparation procedure is unsuitable for heat and/or radiation labile active substances which will be inactivated under the polymerization conditions.

EP-A-0 143 608 also discloses non-rigid collapsible beads as carriers. It further discloses a two-step process in which beads are first formed and then swollen using a swelling agent to allow a solution of a lipophilic compound to be passed into the pores.

It would therefore be desirable to provide for polymeric bead delivery systems comprising relatively rigid polymeric beads which allow for substantially complete release of the active ingredient from the pore network of the beads. It would be particularly desirable if such bead delivery systems could be prepared prior to the introduction of the active substance so that the active substance is not exposed to relatively harsh polymerization conditions.

The present invention provides for a polymeric delivery system for a variety of active substances, such as infrared absorbants (sunscreens), insect repellants, steroids, emollients, and the like, which delivery system may be used alone or may be incorporated into a secondary carrier or vehicle composition, or other cosmetic product. The polymeric delivery system with an incorporated active substance is a dry, free-flowing product which can be rubbed directly on the skin, providing for the controlled release of the substance over time. In the more usual situation where the polymeric delivery system is incorporated in another carrier, vehicle, or cosmetic product, use of the delivery system avoids incompatibilities, typically chemical or physical interactions, which might otherwise exist between the substance and other active ingredient(s) in the cosmetic preparation, or between the active substance and the carrier or vehicle itself.

The controlled release of the active substance provided by the polymeric delivery system of the present invention affords a prolonged activity of the substance on the skin. Such prolonged activity reduces the need to frequently reapply the active substance. Additionally, controlled release both reduces any odor which may be associated with the active substance and lessens the possibility of toxicity and allergic reaction resulting from contact of the active substance with the skin.

According to the present invention, there is provided a delivery system capable of retaining an active substance, said delivery system comprising a plurality of cross-linked polymer beads formed by polymerisation of polyethylenically unsaturated monomers and monoethylenically unsaturated monomers in an immiscible phase in the presence of a porogen followed by extraction of substantially all residual porogen, each bead defining a rigid, substantially non-collapsible pore network which is substantially free from porogen and other retained substances; the beads being substantially free from reactive groups that would interact with an active substance; the beads being adapted to take up active substances into their existing rigid pore network and retain them there, releasably, by capillary action. In a second aspect there is provided a method for preparing a composition containing an active substance for providing delivery thereof, said method comprising:

(a) preparing by polymerisation of polyethylenically unsaturated monomers and monoethylenically unsaturated monomers in an immiscible phase, in the presence of a porogen, a plurality of rigid cross-linked polymer beads wherein the beads have a degree of cross-linking density in the range from 20% to 80% (calculated as the weight of polyethylenically unsaturated monomer (or monomers) divided by the total weight of monomers); the cross-linking density being selected so that each bead defines a substantially non-collapsible internal network of pores capable of retaining the active substance but having residual porogen therein;

(b) extracting substantially all residual porogen from the pore network; and

(c) introducing the active substance in liquid form into the existing network of pores in the rigid beads so that it is held therein by capillary action.

Generally the beads are formed by suspension polymerisation of suitable monomers in an immiscible phase containing a porogen.

Generally the monomers and the porogen are first mixed together and the resulting mixture then suspended in the immiscible phase, usually an aqueous phase. The immiscible phase is then agitated to form droplets of the monomer mixture, and polymerization of the monomer mixture is initiated to form the desired beads from the droplets. Relatively rigid beads having a substantially non-collapsible pore network are formed by providing a cross-linking density in the range from about 20% to 80%, more usually being in the range from 25% to 60% cross-linking, and typically being in the range from about 45% to 55% cross-linking. The bead diameter is normally maintained in the range from about 5 microns to 100 microns, usually being about 10 microns to 50 microns, and the total pore volume is in the range from about 0.1 to 2.0 cc/g, usually being in the range from about 0.3 to 1.0 cc/g. The surface area of the beads will range from about 1 to 500 $m^2$/g, usually being in the range from about 20 to 200 $m^2$/g. The precise dimensions and characteristics of the beads are controlled by varying process parameters such as agitation speed and nature of the porogen.

Once the beads are formed, porogen is extracted from the bead product, typically using solvent extraction or evaporation. The desired active substance may then be introduced into the beads, typically by contact absorption, to create the desired final product. In addition to allowing the incorporation of labile active substances, such a two-step preparation process allows greater control over the structure of the bead resulting from a wider choice of porogens and reaction conditions.

DESCRIPTION OF THE SPECIFIC EMBODIMENTS

Compositions for topical application are formed by incorporating an active substance, such as a UV absorbant, an insect repellant substance, a steroid, an emollient, or the like, inside a polymer delivery system comprising cross-linked polymer particles defining an extensive internal pore network capable of retaining the substances. The pore network is open to the external surface of the bead, allowing controlled release of the active substance over time after the particles are applied to the skin. The particles, usually spherical beads, are formed by suspension polymerization of a monomer (or monomers) and porogen mixture suspended in an immiscible phase, typically an aqueous immiscible phase. The suspension is agitated, causing small droplets of the monomer and porogen mixture to form within the immiscible phase. Polymerization and cross-linking of the monomer(s) creates a non-collapsible bead having an internal pore network defined by the entrapped porogen. The porogen is then extracted from the beads, leaving the open pore network substantially empty and capable of receiving the active substance. The active substance may be introduced to the beads, typically by contact absorption, immediately following extraction of the porogen. Alternatively, the extracted beads may be stored for some time prior to introduction of the active substance, allowing shipment of the beads to another location for final preparation of the desired product.

The polymer delivery system of the present invention comprises rigid polymeric beads having a non-collapsible pore structure. That is, the beads will substantially retain their internal pore structure even after the porogen and/or the active substance has been extracted and the pores are empty. Such beads are mechanically stable compared with non-rigid materials, allowing manufacturing, processing, and handling of the beads under relatively rigorous conditions which might result in the rupture or damage of less stable materials. More importantly, the non-collapsible pores allow substantially complete utilization of the active substance and facilitate introduction of the active substance into the bead after the porogen has been extracted.

The rigid polymeric bead of the present invention is formed by polymerization and cross-linking of one or more preselected monomers to form a molecular structure having a substantially non-collapsible network of pores resulting from the presence of the porogen during polymerization. At least one monomer will be polyethylenically unsaturated, and usually the polymer will include a monoethylenically unsaturated co-monomer. The degree of cross-linking may then be controlled by adjusting the ratio of monoethylenically unsaturated monomer to polyethylenically unsaturated monomer, as discussed in more detail hereinbelow. The active substance is held by capillary action within the network of pores and remains there until an external force or energy draws the substance from the beads. The rigid structure of the bead prevents significant shrinkage or collapse of the bead as the active substance is removed from the network of pores. This is an advantage as it helps attain substantially complete removal and utilization of the active substance. This is contrast to non-rigid beads where the pore structure will collapse as the active substance is extracted, rendering it difficult or impossible for the substance which is entrapped deep within the pore structure to be removed and utilized.

The primary difference between the formation of non-rigid beads and rigid beads of the present invention is the degree of cross-linking imparted to the polymer. The rigid polymer beads of the present invention will have from about 20% to 80% cross-linking, more usually having in the range from about 25% to 60% cross-linking, and typically being in the range from about 45% to 55% cross-linking. The calculated or theoretical percentage of cross-linking is defined as the weight of polyethylenically unsaturated monomer (or monomers) divided by the total weight of monomers, including both polyethylenically unsaturated and monoethylenically unsaturated monomers.

The beads of the polymer are conveniently formed by suspension polymerization in a liquid-liquid system. In general, a solution containing monomers, a polymerization catalyst (if used), and an inert but fully miscible liquid porogen is formed which is immiscible with water. The solution is then suspended in an aqueous solution, which generally contains additives such as surfactants and dispersants to promote the suspension. Once the suspension is established with discrete droplets of the desired size, polymerization is effected (typically by activating the reactants by either increased temperature or irradiation). Once polymerization is complete, the resulting rigid beads are recovered from the suspension. The beads at this point are solid porous structures, the polymer having formed around the inert, water-immiscible liquid, thereby forming the pore network. The liquid porogen has accordingly served as a "pore-forming agent" and occupies the pores of the formed beads.

Materials suitable as porogens will be liquid substances which meet the following criteria:

1. They are either fully miscible with the monomer mixture or capable of being made fully miscible by the addition of a minor amount of non-water-miscible solvent;

2. They are immiscible with water, or at most only slightly soluble;

3. They are inert with respect to the monomers, and stable when in contact with any polymerization catalyst used and when subjected to any conditions needed to induce polymerization (such as temperature and radiation); and

4. They are readily extracted from the pore network of the beads once polymerization is complete.

Suitable porogens include a wide range of substances, notably inert, non-polar organic solvents. Some of the most convenient examples are alkanes, cycloalkanes, and aromatics. Specific examples of such solvents are alkanes of from 5 to 12 carbon atoms, straight or branched chain cycloalkanes of from 5 to 8 carbon atoms, benzene, and alkyl-substituted benzenes, such as toluene and the xylenes. Extraction of the porogen may be effected by solvent extraction, evaporation, or similar conventional operations. The porogen extraction step accomplishes the removal of unwanted species from the polymerized structures prior to impregnation with the desired active substance. Such unwanted species include unreacted monomers, residual catalysts, and surface active agents and/or dispersants remaining on the bead surfaces.

Extraction of the porogen and its replacement with (i.e., impregnation of the dry bead with) the above substance in the above-described procedure may be effected in a variety of ways, depending on the chemical nature of the porogen and its behavior in combination with that of the other species present. For example, the beads may be recovered from the suspension by filtration, preferably using vacuum apparatus (such as a Beuchner funnel). The beads are then washed with an appropriate solvent to remove organic species not bound to the polymer, including surfactants having deposited on the bead surfaces from the aqueous phase, unreacted monomers and residual catalysts, and the porogen itself. An example of such a solvent is isopropanol, either alone or in aqueous solution. Once washing is complete, the solvent itself is removed by drying, preferably in a vacuum.

4

In certain cases, an alternative method of extraction may be used - i.e., where the porogen, unreacted monomer and water will form an azeotrope. In these cases, steam distillation is an effective way of extracting porogen from the beads. This again may be followed by drying under vacuum.

Once the beads are rendered dry and free of the porogen and any unwanted organic materials, they are impregnated with the active substance according to conventional techniques. The most convenient such technique is contact absorption, aided by solvents if necessary to enhance the absorption rate.

The polymerization process used in preparing the beads of the polymer delivery system can be modified to control both the porosity and the particle diameter of the beads. Controlling the porosity, in turn, controls the rate at which the active material will be absorbed and/or released from the beads. Particle diameter is controlled primarily by the degree of agitation, with more rigorous agitation causing smaller droplets and hence smaller polymerized beads. The pore diameter and pore volume, in contrast, are controlled primarily by the cross-linking density. Porosity is increased by increasing the amount of cross-linking monomer used, or by increasing the porogen concentration in the monomer mixture, or both. An increase in porosity increases the surface area of the bead and hence the weight percent of the active substance which may be held within the bead. Bead diameter is also affected by the concentration of dispersing agent in the immiscible phase.

The bead diameter in the polymer delivery system should be in the range from about 5 to 100 microns. Beads having an average diameter in the range from about 5 microns to no more than about 70 microns are preferred, with a bead diameter in the range from about 10 microns to about 40 microns being particularly preferred. Beads with a diameter from 10 to 40 microns have been found to be aesthetically pleasing when topically applied to the skin.

The pore dimensions within the beads may vary widely, with optimum dimensions depending on the chemical characteristics of the polymers used as well as the diffusive characteristics of the active substance. Different systems will thus call for different optimum ranges of pore volume distribution to obtain the most desirable properties for the overall formulation. In general, however, best results are obtained with total pore volumes ranging from about 0.1 to about 2.0 cc/g, preferably from about 0.3 to about 1.0 cc/g; pore surface areas ranging from about 1 to about 500 $m^2/g$, preferably from about 20 to about 200 $m^2/g$; and average pore diameters ranging from about 0.001 to about 3.0 microns, preferably from about 0.003 to about 1.0 micron. Following conventional methods of measuring and expressing pore sizes, the pore diameters are measured by techniques such as nitrogen or mercury porosimetry and are based on the model of a pore of cylindrical shape.

In order to form the cross-linked polymer beads of the present invention, it is necessary to polymerize either polyethylenically unsaturated monomers, i.e., those having at least two sites of unsaturation, or to polymerize monoethylenically unsaturated monomers in the presence of one or more polyethylenically unsaturated monomers. In the latter case, the percentage of cross-linking may be controlled by balancing the relative amounts of monoethylenically unsaturated monomer and polyethylenically unsaturated monomer.

Monoethylenically unsaturated monomers suitable for preparing polymer beads for the polymer delivery system include ethylene, propylene, isobutylene, diisobutylene, styrene, ethylvinylbenzene, vinyltoluene, and dicyclopentadiene; esters of acrylic and methacrylic acid, including the methyl, ethyl, propyl, isopropyl, butyl, sec-butyl, tert-butyl, amyl, hexyl, octyl, ethylhexyl, decyl, dodecyl, cyclohexyl, isobornyl, phenyl, benzyl, alkylphenyl, ethoxymethyl, ethoxyethyl, ethoxypropyl, propoxymethyl, propoxyethyl, propoxypropyl, ethoxyphenyl, ethoxybenzyl, and ethoxycyclohexyl esters; vinyl esters, including vinyl acetate, vinyl propionate, vinyl butyrate and vinyl laurate; vinyl ketones, including vinyl methyl ketone, vinyl ethyl detone, vinyl isopropyl ketone, and methyl isopropenyl ketone; vinyl ethers, including vinyl methyl ether, vinyl ethyl ether, vinyl propyl ether, and vinyl isobutyl ether; and the like.

Polyethylenically unsaturated monomers which ordinarily act as though they have only one unsaturated group, such as isopropene, butadiene and chloroprene, may be used as part of the monoethylenically unsaturated monomer content.

Polyethylenically unsaturated cross-linking monomers suitable for preparing such polymer beads include diallyl phthalate, ethylene glycol diacrylate, ethylene glycol dimethacrylate, trimethylolpropanetrimethacrylate, divinylsulfone; polyvinyl and polyallyl ethers of ethylene glycol, of glycerol, of pentaerythritol, of diethyleneglycol, of monothio- and dithio-derivatives of glycols, and of resorcinol; divinylketone, divinylsulfide, allyl acrylate, diallyl maleate, diallyl fumarate, diallyl succinate, diallyl carbonate, diallyl malonate, diallyl oxalate, diallyl adipate, diallyl sebacate, divinyl sebacate, diallyl tartrate, diallyl silicate, triallyl tricarballylate, triallyl aconitate, triallyl citrate, triallyl phosphate, divinyl naphthalene, divinylbenzene, trivinylbenzene; alkyldivinylbenzenes having from 1 to 4 alkyl groups of 1 to 2 carbon atoms substituted on the benzene nucleus; alkyltrivinylbenzenes having 1 to 3 alkyl groups of 1 to 2 carbon

EP 0 277 211 B1

atoms substituted on the benzene nucleus; trivinylnaphthalenes, and polyvinylanthracenes.

The preferred polymer bead of the present invention will be free from reactive groups which will interact with the porogen and/or the active substance which is ultimately incorporated in the composition. In particular, the beads should be free from reactive amino, hydroxyl, carboxylic, and other reactive functionalities. Such beads will not readily undergo unwanted reactions, will be stable over a wide pH range, will resist moderate oxidation and reduction, will be stable at higher temperatures, will resist attack by moisture, and will have a relatively long shelf life.

The particularly preferred polymer delivery system of the present invention is formed by the copolymerization of styrene and divinylbenzene, vinyl stearate and divinylbenzene, or methylmethacrylate and ethylene glycol dimethylmethacrylate. Usually, the monoethylenically unsaturated monomer will be present at about 20 to 60 percent of the monomer mixture, typically being in the range from about 45 to 55 percent of the monomer mixture, with the polyethylenically unsaturated monomer forming the remainder of the mixture. Particularly preferred is the styrene-divinylbenzene polymeric bead which consists essentially of a hydrocarbon backbone with benzene rings and which is substantially completely free from reactive groups.

Exemplary active substances which may be introduced to the polymer bead delivery system of the present invention include ultraviolet (UV) absorptive materials which form a sunscreen product, insect repellant substances, steroids, emollients, and the like.

UV absorptive materials suitable for the present invention will be solids or liquids capable in pure form of absorbing at least 95% of the ultraviolet radiation at wavelengths in the range from about 290 to 320 nm, the radiation primarily responsible for causing sunburn. The materials may transmit some or all UV radiation above 320 nm, particularly if tanning is desired. The presently known UV absorptive materials which are accepted as safe for human use may be classified into five groups, as set forth in Table 1.

6

TABLE 1

| Group | Exemplary Compounds | Absorbance |
|---|---|---|
| Aminobenzoates | p-Aminobenzoic acid (PABA) | 260-313 nm |
| | Ethyl 4-[bis(hydroxypropyl)] aminobenzoate | 280-330 nm |
| | Octyl dimethyl PABA | - - - - - - - - - |
| | PABA propoxylate | - - - - - - - - - |
| | Glyceral PABA | 264-315 nm |
| | 2-Ethylhexyl PABA (Padimate O) | - - - - - - - - - |
| | Pentyl PABA (Padimate A) | - - - - - - - - - |
| Cinnamates | Cinoxate | 270-328 nm |
| | Diethanolamine p-methoxy cinnamate | 280-310 nm |
| | 2-Ethylhexyl p-methoxycinnamate | 290-320 nm |
| Benzones | Dioxybenzone | 260-320 nm |
| | Sulisobenzone | - - - - - - - - - |
| | Oxybenzone | 270-350 nm |
| | 2-Ethylhexyl 2-cyano-3,3-diphenylacrylate | - - - - - - - - - |
| Salicylates | 2-Ethylhexyl salicylate | 280-320 nm |
| | Triethanol amine salicylate | 260-320 nm |
| | Homosalate | 295-315 nm |

| Miscellaneous | Red petrolatum | --------- |
| | Titanium dioxide | --------- |
| | Digalloyl trioleate | 270-320 nm |
| | Lawsone with dihydroxyacetone | 290-400 nm |
| | 2-Phenylbenzimidazole-5-<br>sulfonic acid | 290-320 nm |

The UV absorptive substances listed in Table 1 may be used alone or in mixtures of two or more when it is desired to increase the range of UV absorption over that offered by any one substance. When combining UV absorptive substances, care should be taken to avoid undesirable interactions between the substances.

Surprisingly, it has been found that sunscreen compositions prepared by introducing a UV absorptive substance into polymeric beads prepared by the method of the present invention results in a composition capable of adsorbing infrared radiation as well as ultraviolet radiation. As the dangers of exposure to infrared radiation become more apparent, the utility of a sunscreen which is able to absorb both infrared and ultraviolet radiation becomes increasingly clear.

Insect repellant substances suitable for incorporation into the compositions of the present invention will function through volatilization and formation of a thin protective barrier or layer as the repellant is released from the polymer delivery system. The repellant substances will usually be liquids, although solids which are dissolved or dispersed in a liquid carrier may also find use. The substances should be generally non-toxic, at least when incorporated in the polymer delivery system, and should be effective against a wide variety of insects. Insect repellant substances which are presently accepted as safe and which are suitable for use in the present invention are set forth in Table 2.

TABLE 2

| Group | Exemplary Compounds |
|---|---|
| Terpenoids | Citronellal<br>Geraniol<br>Terpentine<br>Pennyroyal<br>Cedarwood<br>Eucalyptus<br>Wintergreen |
| Benzoquinones | Benzquinone and its homologs, methyl ether derivatives and homologs |
| Aromatics | Cresols<br>Benzaldehyde<br>Benzoic acids |
| Synthetics | N,N-diethyl-m-toluamide (deet)<br>Ethyl hexanediol<br>Dimethyl phthalate<br>Dimethyl ethyl hexanediol carbate<br>Butopyronoxyl<br>Di-n-propyl isocinchonmeronate<br>N-Octyl bicycloheptene dicarboximide<br>2,3,4,5-bis(2-butylene)tetra-hydro-2-furaldehyde |

The insect repellant substances listed in Table 2 may be used alone, or more desirably, in combinations tailored to be effective against a greater variety of insects than a single repellant alone. Generally, it will be easier to combine different insect repellant substances inside the polymer delivery system of the present invention than it would be combining them by themselves or in liquid vehicles or carriers. Insect repellant substances which would tend to separate because of physical differences, e.g., immiscibility, may be held within the polymer delivery system in a dispersion or mixture which helps assure that they will be released at substantially the same rate over time.

Suitable emollients include mineral oil, isopropyl myristate, isopropyl palmitate, propoxypropylene myristyl ether propionate, $C_{12}$-$C_{15}$ alcohol benzoates, vegetable oils, e.g., safflower oil, peanut oil or corn oil, silicone oils such as polydimethylcyclosiloxane, hexamethyldisiloxane, dimethicone, amodimethicone, trimethylsilylamodimethicone, stearyl dimethicone, cetyl dimethicone, stearoxy dimethicone, polysiloxane/polyalkyl copolymers, dialkoxydimethylpolysiloxanes, dimethicone copolymers, cetyl dimethicone copolymers and dimethicone propyl PG-betaine, perfluoropolyethers, marine oils, such as shark liver and fish liver oils, linolin, glycerol, sorbitol, bath oils, etc.

Suitable steroids will be adrenocortical steroids, such as fluocinolone, fluocinolone acetonide, triamcinolone acetonide, beta-methasone valerate, timobesone acetate, hydrocortisone, hydrocortisone acetate, triamcinolone, prednisolone, prednisolone acetate, dexamethasone, beclomethasone dipropionate, betamethasone dipropionate, betamethasone benzoate, clocorolone pivalate, halcinonide, flumethasone pivalate, and desonide. A number of anti-inflammatory steroids suitable for use in the present invention have been disclosed in U.S. Patent Nos. 4,017,615; 3,365,446; 3,067,194; 3,364,203; 3,053,833; and 3,513,162.

The polymer delivery composition of the present invention may be incorporated in a suitable carrier or vehicle, or into cosmetic preparations, such as face creams, lipsticks, lip balms, baby creams, lotions, shampoos, after shave lotions, hair grooming preparations, and the like. Alternatively, the compositions, which are dry, free-flowing powders, may be utilized by themselves without further incorporation in a carrier of any kind. Usually, the active substance will comprise from about 5% to 65% of the polymeric composition by weight, more usually comprising from about 20% to 60% by weight, and most often being in the range from about 40% to 55% by weight.

The polymeric beads prepared as just described function as a reservoir for controlled delivery of the active substances providing a wide range of advantages over the conventional formulations. Release of the active substances from the pores occurs in sustained manner, providing a continuous fresh supply of active substance to the epidermal area to which the preparation has been applied. The active substances diffuse out of the pores into either the vehicle if one is used or the natural bodily secretions present on one's skin at the applied area, in accordance with known principles of the diffusion of one liquid through another. The activity-time curve of the active substances are thus extended and flattened out. The magnitude of the release rate is controlled by the pore volume distribution in the bead itself, notably the total pore volume and the average pore diameter. Selection of the values of these parameters according to predetermined standard provides control of the release rate to desired levels.

The following examples are offered by way of illustration, not by way of limitation.


EXPERIMENTAL


Example I


(Preparation of Beads)


A two-liter four-necked reaction flask equipped with a stirrer driven by a variable speed motor, reflux condenser, thermometer, and nitrogen-inlet tube was set up. A slow flow of nitrogen was maintained through the reaction flask at all times. An aqueous phase made up at 350 parts of deionized water, 1.8 parts of gum arabic, and 1.8 parts sodium lignosulfate (Marasperse N-22, available from Reed Lignin, Inc.) was added to the flask, and an organic solution made up 39.65 parts of styrene, 47.60 parts of commercial divinylbenzene (55.6% divinylbenzene, 42.3% ethylvinylbenzene), 71.35 parts of heptane, and 2.2 parts benzoyl peroxide (70% active ingredient and 30% water) was dispersed in the aqueous phase with rapid agitation (stirrer speed approximately 950 rpm) to obtain a plurality of droplets having an average droplet diameter of below about 60 microns as determined by visual observation of a sample of the droplets with an optical microscope.

The reaction mixture was then heated to about 75°C and maintained at that temperature for 10 hours to form porous beads of cross-linked styrene/divinylbenzene copolymer having heptane entrapped within the pores. The reaction mixture was then cooled to room temperature and the resulting polymeric beads collected by filtration, washed three times with 1000 parts of deionized water, and three times with 1000 parts of acetone, then dried in a vacuum oven at 80°C for 24 hours.

The calculated or theoretical cross-link density of the purified beads is 30.3%. This density is calculated by multiplying the weight of divinylbenzene (47.6 g) by the purity of the divinylbenzene (0.556) to get the actual weight of pure divinylbenzene which is then divided by the total weight of monomer (87.25 g).

The surface area of a sample of the purified beads was 146.2m$^2$/g as measured by B.E.T. nitrogen multipoint analysis and the pore volume was 0.99 ml/g as measured by Mercury porosimetry.

Example II

(Preparation of Beads)

A two-liter-necked reaction flask equipped as described in Example I was evacuated and purged with nitrogen. An aqueous phase made up of 450 parts of deionized water, 4 parts of gum arabic, and 4 parts of sodium lignosulfate was added to the flask, and an organic solution made up 52 parts of methylmethacrylate, 78 parts ethyleneglycol dimethacrylate, 1.5 parts of benzoyl peroxide (70% in water), and 150 parts of toluene was dispersed in the aqueous phase with rapid (stirrer speed approximately 900 rpm) to obtain a plurality of droplets having an average droplet of below about 60 microns, as determined by visual observation of a sample of the droplets being stabilized by the dispersants.

The reaction mixture was heated to 65°C for 1 hour, then 75°C and allowed to remain at this temperature for approximately 7 hours while maintaining a nitrogen flow of 2 ml/minute to form porous beads of cross-linked methacrylate/ethyleneglycoldimethacrylate copolymer having toluene entrapped within the pores. The reaction mixture was then cooled and the beads collected by filtration, washed three times with 1000 parts of deionized water, and three times with 1000 parts of acetone, then dried in a vacuum oven at 80°C for about 24 hours.

The calculation of theoretical cross-linking density of the purified beads is 60% and is calculated by dividing the weight of ethyleneglycoldimethacrylate (78 g) by the weight of monomer (130 g).

The surface area of a sample was 180.59 m$^2$/g and the pore volume was 0.684 ml/g, determined as described in Example I above.

Example III

(Impregnation of Beads of Example I with UV Absorbant)

A 25 parts portion of macroporous cross-linked copolymer beads as described in Example I above was mixed at room temperature with 100 parts of isopropanol in a glass beaker with a stirring bar. Then 25 parts of octyl dimethyl PABA were added slowly with stirring. The solvent was then allowed to evaporate to dryness in a fume hood at room temperature. The beads containing 49.7% octyl dimethyl PABA entrapped within their pores are obtained.

Example IV

(Impregnation of Beads of Example I with UV Absorbant)

By repeating the procedure of Example III, using 25 parts of the styrene/divinylbenzene porous cross-linked polymeric beads prepared in Example I, 25 parts of 2-ethylhexyl-p-methoxycinnate and 100 parts of isopropanol as the solvent, beads containing 49.0% 2-ethylhexyl-p-methoxycinnanamate entrapped within their pores are obtained.

Example V

(Impregnation of Beads of Example I with UV Absorbant)

By again repeating the procedure of Example IV, using 50 parts of the methylmethacrylate/ethyleneglycoldimethacrylate polymeric beads prepared by Example II, 50 parts of a mixture of octyldimethyl PABA and oxybenzone-3 (7 parts of octyldimethyl PABA and 3 parts of oxybenzone-3), and 140 parts of isopropanol as the solvent, beads containing 49.6% octyldimethyl PABA/oxybenzone-3, entrapped within their pores are obtained.

Example VI

(Preparation of Beads)

A 2000 ml four-necked reaction flask equipped with a motorized stirrer, reflux condenser, thermometer, and nitrogen inlet was evacuated and purged with nitrogen. 900 Parts of deionized water, 7.2 parts of gum arabic and 7.2 parts of a sodium-based lignosulfonate (Reed lignin) available from the American Can Co. under the trademark Marasperse N-22, were charged to the reaction flask. The mixture was heated, with stirring, in an oil bath at about 50 degrees Celsius until the dispersants (gum arabic and lignosulfate) dissolved to form an aqueous phase.

To this mixture there was then added a freshly prepared solution of 85.6 parts of styrene (99.8% purity), 102.3 parts of commercial divinylbenzene (55.6% divinyl benzene, 42.3% ethylvinylbenzene), 7.7 parts of benzoyl peroxide (70% active ingredient and 30% water), and 188 parts of toluene (porogen). The aqueous phase and organic solution were agitated by stirring at a rate adjusted to give a plurality of droplets having an average droplet diameter of about 10-60 microns, as determined by visual observation of a sample of the droplets with an optical microscope (400X) with the droplets being stabilized by the dispersants. The reaction mixture was then heated to about 95 degrees Celsius and maintained at that temperature for about 20 hours, at the previously adjusted stirring rate, to form porous beads of cross-linked styrene/divinylbenzene copolymer having toluene entrapped within the network of pores. The mixture was then cooled and the porous polymeric beads were removed from the reaction flask by filtration. The filtered beads were washed initially three times with one liter portions of deionized water to remove the dispersants, followed by three washes with one liter portions of isopropanol to remove any residual, unreacted monomer and the toluene used as the porogen during polymerization. The beads were then dried in an oven at 70°C for six hours.

Macroporous cross-linked polymer beads having the following characteristics were obtained:

| | |
|---|---|
| Styrene, parts | 85.6 |
| Divinylbenzene, parts | 102.3 |
| Porogen, parts Toluene, | 188 |
| Calculated Cross-Linking Density, % | 30 |
| Average Particle Diameter, $\mu$m | 25 |
| Surface Area, $M^2$/g | 1.8 |
| Pore Volume, ml/g | 0.04 |

Example VII

(Insect Repellant)

A 15 part portion of the macroporous cross-linked polymer beads prepared as described in Example VI above was mixed at room temperature with a 60 part portion of diethyl-m-toluamide, and the resulting suspension was stirred at about 100 rpm for 24 hours in a closed container.

The suspension was then filtered and the filtrate washed three times with an aqueous detergent solution (Triton), then three times with deionized water. The washed beads were then oven-dried at 70°C for 6 hours, and their diethyl-m-toluamide contents were determined by acetone extraction to be 45%.

Example VIII

(Release of Insect Repellant from Beads)

A 0.5 part sample of the diethyl-m-toluamide containing beads of Example VII, on a sheet of filter paper, and a sheet of filter paper impregnated with an equivalent amount of diethyl-m-toluamide, were heated under a vacuum of 25 inches of mercury at 100°C for 10 hours, during which time the percentage weight loss of diethyl-m-toluamide was determined each hour by weighing the bead and filter paper samples. The results of these weight loss determinations demonstrate that a high degree of sustained release can be achieved using the polymeric delivery systems of this invention.

Example IX

(Steroid)

A 2000 ml four-necked reaction flask equipped with a stirrer, condenser, thermometer, and nitrogen inlet was evacuated and charged with nitrogen. 800 ml deionized water, 6.4 grams of gum arabic and 6.4 grams of a lignosulfonate available from the American Can Co. under the trademark Marasperse N-22, were charged into the reaction flask. The mixture was stirred for about 30 minutes. To this mixture was added a freshly prepared solution of 85.6 grams of styrene (99.8% purity), 102.3 grams commercial divinylbenzene (55% divinylbenzene), 5.33 grams benzoyl peroxide (70% active ingredient and 30% water), and 187.9 grams of toluene to serve as a porogen. The phase and solution were agitated by a mechanical stirrer whose stirring rate of about 900-1200 rpm was adjusted to obtain a plurality of droplets having a droplet diameter smaller than about 50 microns. The gum arabic and lignosulfonate serve to stabilize the plurality of droplets. The reaction mixture was heated to about 78 degrees Celsius while maintaining a constant rate of stirring and passing a slow stream of nitrogen through the reaction vessel. After about 2 hours cross-linking became noticeable. The mixture was stirred another 22 hours at 78°C and was then allowed to cool to room temperature. The porous polymeric beads were removed from the reaction flask by filtration and washed several times with water to remove gum arabic and lignosulfonate, followed by several washes of isopropanol/acetone mixed solvent (7:3 by volume) and were finally stirred in 400 ml of isopropanol/acetone mixed solvent (7:3) for 20 hours. The polymer was filtered and dried overnight at 65°C in vacuo. The yield was practically quantitative. The residual monomers such as styrene, DVB and naphthalene were smaller than about 0.01%.

The calculated or theoretical cross-linking density of the purified beads is 30%. This density is calculated by multiplying the weight of divinylbenzene (102.3 g) by the purity of the divinylbenzene (.55) to get the actual weight of pure divinylbenzene which is then divided by the total weight of monomer (102.3 g + 85.6 g).

The surface area of a sample of the purified beads was determined by the B.E.T. method to be 1.8 meters$^2$/gram. The B.E.T. method is described in detail in Brunauer, S. Emmet, P.H., and Teller, E., J. Am. Chem. Soc., 60, 309-16 (1938). The surface area of the polymer can be modified by using different porogens such as stable oil compounds which might include, by way of example only, mineral oil, vegetable oils or silicon oils.

The particle size of the beads was determined by an optical microscope to be 60 microns or less with an average approximate particle size diameter of about 10 microns.

The adrenocortical steroid fluocinonide (Syntex) was entrapped in the beads described above by exposing the beads to a 1% solution of fluocinonide in propylene carbonate : propylene glycol (7:3) for a period of time sufficient to allow the beads to absorb the active ingredient solution. The amount of active ingredient solution used relative to the amount of polymer beads was adjusted according to the desired final concentration of active ingredient to be contained within the beads. Where a low final concentration is desired, the active ingredient solution may be further diluted with a solvent such as acetone, methanol or ethanol prior to combining the solution with the beads in order to achieve a sufficient amount of starting solution to form a slurry with the beads. The diluent solvent was later removed by heating under a vacuum.

To obtain beads with a final active ingredient concentration of 0.05%, 7.6 g of the polymer beads described above were combined with 0.4 g of a 1% solution of fluocinonide in propylene carbonate : propylene glycol (7:3) and 14.8 g acetone. The initial slurry was stirred approximately every five minutes over a period of approximately thirty minutes, during which period the mixture becomes cake-like and, finally, powder-like in consistency. The resulting powder was then oven-dried for approximately three hours at 40 - 60°C and 25 mmHg, at which point the powder reached a constant weight and the acetone was removed. Similarly, a 0.25% formulation may be achieved by mixing 4.8 g polymer beads, 3.2 g steroid solution and 6.4 g acetone as described above.

It has been found that the therapeutic anti-inflammatory activity of fluocinonide-containing beads in a petrolatum-based delivery medium is comparable, based on final weight concentration of the fluocinonide, to that of commercially-available fluocinonide ointments such as Lidex™ (Syntex). Thus, ointments formed using the delivery vehicles of the present invention may employ active ingredient concentration parallel to those of typical ointments, i.e., 0.01% to 1% by weight. It should be noted, however, that therapeutically effective anti-inflammatory compositions may include as little as 0.00001% by weight steroid active ingredient and as much as 5% by weight steroid or higher. A range of 0.01% to 0.2% is particularly useful, with 0.01% to 0.05% being preferred for the more active corticosteroids such as the fluocinonides, and 0.01% to 0.1% being preferred for less active corticosteroids such as the betnovates and the triamcinolones. When polymer beads containing active ingredient are used topically in powder form, therapeutic anti-inflammatory activity may be lower than that of commercial ointments, although activity is increased and/or provided over longer time periods if the polymer beads are rubbed occasionally to promote release of the active ingredient.

Suitable ointments containing polymer beads with active ingredient were prepared by combining an appropriate amount of the polymer beads with petrolatum and an emulsifier (Amerchol CAB). To achieve a 0.05% fluocinonide ointment, 4.6 parts by weight (pbw) Amerchol CAB, 32.2 pbw white petrolatum USP (Ultima) and 50.7 pbw white petrolatum USP were first combined and melted, and 12.5 pbw 0.4% fluocinonide polymer beads was then mixed with the melted mixture and cooled. A 0.1% ointment was obtained by combining 4.0 pbw Amerchol CAB, 28.4 pbw white petrolatum USP (Ultima) and 42.6 pbw white petrolatum (USP) with 25.0 pbw 0.4% fluocinonide beads. A 0.2% ointment was formulated by combining 2.6 pbw Amerchol CAB, 19.0 pbw white petrolatum (USP) and 28.4 pbw white petrolatum USP with 50.0 pbw 0.4% fluocinonide beads. By starting with polymer beads containing different amounts of active ingredient, the relative weight proportion of polymer bead delivery vehicle can be modulated.

The efficacy of the polymer bead delivery vehicle of the present invention was demonstrated for both the powder and ointment forms of the beads using a vasoconstriction assay. This method is based on the Stoughton-McKenzie vasocontriction assay for corticosteroid formulations (McKenzie, A.W., and Stoughton, R.B., "Method for Comparing Percutaneous Absorption of Steroids," Arch. Dermatol., 86, 608-10 (1962)). All test preparations was placed in identical containers, coded and assigned by random tables to individual test sites. The test subjects are normal adult male and female volunteers not receiving any steroids and who have not participated in any studies using steroids for at least four weeks prior to testing. The forearms of the subjects are prepared by gentle washing and drying. Strips of double-adhesive coated Blenderm™ tape (3M) with 7 x 7 mm prepunched squares are applied to each forearm to isolate the application sites. An appropriate dose of the test formulation (either 2 mg or 3 mg) was then applied to the skin in each square and was spread and rubbed with consistent pressure using a clean HPLC vial at each application site. In cases where powder-form polymer beads containing fluocinonide is used, the forearm was inverted after application and each individual site was gently brushed with a clean square of gauze to remove excess polymer beads. A protective cage was applied over the sites on the forearm designated for "open" application. On the other arm ("occluded") the sites were covered with Saran Wrap™, the margins sealed with tape and a protective cage placed over the sites. After six hours of exposure of the skin to the corticosteroid preparations, all the tapes are removed and the forearms are washed.

Scoring in the assay was preformed by two experienced observers who independently scored the presence or absence of vasoconstriction and the degree and blanching at 8, 24 and 32 hours after the time of application of the formulations to the sites.

As evidenced in Table 3, the powder form polymer bead formulations of the present invention achieved significant vasoconstriction as compared to commercially-supplied Lidex™ fluocinonide ointment (Syntex) not using a polymer bead delivery vehicle. Although vasoconstriction due to the powder form polymer beads is somewhat less than that observed with the Lidex™ ointment, this difference may be due to the fact that excess powder formulation is brushed off after application to the forearms. Table 4 demonstrates that intermittent rubbing of the powder-form formulations acts to promote and prolong vasoconstriction activity. Table 5 demonstrates that the polymer bead delivery vehicle of the present invention, when applied in an ointment form comparable to that of commercially-supplied fluocinonide ointment, achieves a level of vasoconstriction approximately equal to that of the commercially-supplied product. This effect is achieved independent of any rubbing of the polymer bead ointment subsequent to application. It may be expected that such rubbing will further enhance vasoconstriction activity attributable to the delivery vehicle of the present invention.

TABLE 3

| Vasoconstriction Assay Readings--Polymer Powder Formulations | | | | |
|---|---|---|---|---|
| FLUOCINONIDE FORMULATION | Hours After Application | | | Total % |
| | 8 | 24 | 32 | |
| Polymer Beads (0.4%) Occluded Application: | | | | |
| Sites Responding (%): Intensity of Response (%): | 50.0 22.9 | 62.5 22.9 | 50.0 16.7 | 162.5 62.5 |
| Polymer Beads (0.4%) Open Application: | | | | |
| Sites Responding (%): Intensity of Response (%): | 62.5 25.0 | 56.3 18.8 | 25.0 8.3 | 143.8 52.1 |
| Ointment (0.05%) Occluded Application: | | | | |
| Sites Responding (%): Intensity of Response (%): | 100.0 72.9 | 87.5 33.3 | 75.0 27.1 | 262.5 133.3 |
| Ointment (0.05%) Open Application: | | | | |
| Sites Responding (%): Intensity of Response (%): | 93.8 68.8 | 100.0 39.6 | 93.8 31.3 | 287.6 139.7 |
| NOTE: Dosage was 2 mg of polymer powder, with entrapped fluocinonide (0.4%), or 2 mg Lidexz 0.05% fluocinonide ointment. Test sites were rubbed at time zero, washed at time 6 hours, and read at the times indicated. | | | | |

TABLE 4

| Effect of Re-Rubbing on Vasoconstriction Effect of Polymer Powder Formulations | | | | |
|---|---|---|---|---|
| FLUOCINONIDE FORMULATION | Hours After Application | | | Total % |
| | 8 | 24 | 32 | |
| Polymer Beads (0.5%) | | | | |
| Sites Responding (Increase %) Intensity of Response (Increase %) | 25.0 8.3 | 12.5 4.1 | 31.3 10.4 | 62.8 22.8 |
| Polymer Beads (0.25%) | | | | |
| Sites Responding (Increase %) Intensity of Response (Increase %) | 0 0 | 31.3 10.4 | 31.3 12.5 | 62.6 22.9 |
| Polymer Beads (0.4%) | | | | |
| Sites Responding (Increase %) Intensity of Response (Increase %) | -18.7 -10.4 | 12.5 4.1 | 25.0 10.4 | 18.8 4.1 |
| NOTE: Dosage was 2 mg of polymer powder, with entrapped fluocinonide at indicated proportion. All test sites were left open (unoccluded) and were rubbed and brushed off at time zero. Control powder sites were washed at time 6 hours; re-rubbed powder sites were re-rubbed at 6, 8, and 24 hours. Readings were made at times indicated. Data represents percent readings taken at re-rubbed sites minus percent readings taken at corresponding control sites. | | | | |

TABLE 5

Vasoconstriction Assay Readings--
Polymer-in-Ointment Formulations

|  | Hours After Application | | | |
|---|---|---|---|---|
|  | 8 | 24 | 32 | Total % |
| FLUOCINONIDE FORMULATION | | | | |
| **Polymer Beads (0.05% in Ointment-- Occluded)** | | | | |
| Sites Responding (%) | 87.5 | 68.8 | 81.3 | 237.6 |
| Intensity of Response (%) | 75.0 | 29.2 | 29.2 | 133.4 |
| **Polymer Beads (0.05% in Ointment--Open)** | | | | |
| Sites Responding (%) | 87.5 | 62.5 | 68.8 | 218.8 |
| Intensity of Response (%) | 72.9 | 20.8 | 25.0 | 118.7 |
| **Polymer Beads (0.1% in Ointment--Occluded)** | | | | |
| Sites Responding (%) | 87.5 | 62.5 | 68.8 | 218.8 |
| Intensity of Response (%) | 66.7 | 25.0 | 22.9 | 114.6 |
| **Polymer Beads (0.1% in Ointment--Open)** | | | | |
| Sites Responding (%) | 93.8 | 75.0 | 81.3 | 250.1 |
| Intensity of Response (%) | 72.9 | 27.1 | 29.2 | 129.2 |
| **Polymer Beads (0.2% in Ointment--Occluded)** | | | | |
| Sites Responding (%) | 75.0 | 81.3 | 81.3 | 237.6 |
| Intensity of Response (%) | 58.3 | 29.2 | 29.2 | 116.7 |
| **Polymer Beads (0.2% in Ointment--Open)** | | | | |
| Sites Responding (%) | 93.8 | 62.5 | 37.5 | 193.8 |
| Intensity of Response (%) | 68.8 | 25.0 | 12.5 | 106.3 |
| **Commercial Ointment (0.05%--Occluded)** | | | | |
| Sites Responding (%) | 93.8 | 68.8 | 68.8 | 231.4 |
| Intensity of Response (%) | 79.2 | 27.1 | 22.9 | 129.2 |
| **Commercial Ointment (0.05%--Open)** | | | | |
| Sites Responding (%) | 87.5 | 75.0 | 87.5 | 250.0 |
| Intensity of Response (%) | 77.1 | 27.1 | 33.3 | 137.5 |

NOTE: Dosage was 3 mg of petrolatum-based ointment containing poly-
mer powder, with entrapped fluocinonide at indicated proportion, or
3 mg Lidex™ 0.05% fluocinonide ointment. Test sites were rubbed at
time zero, washed at time 6 hours, and read at the times indicated.

In an additional study, fluocinonide was dissolved in a 30/70 propylene glycol/propylene carbonate system and entrapped in the polymer delivery system of the present invention. The degree of vasoconstriction produced served as an indicator of the release of the corticosteroid solution from the delivery system.

Equal amounts of the beads were directly applied to human forearms, rubbed gently, and the excess powder brushed off. On one arm, no further application or manipulation was made. On the other arm, the site of initial application was gently rubbed at 7, 23, and 31 hours, but no additional product was added.

Vasoconstriction responses were measured and recorded at 8, 24, and 32 hours and the results are presented in Table 6. The increased and continued vasoconstriction produced in the arm that was rubbed several times is definitive evidence of the demand and sustained release of the corticosteroid solution from the polymer delivery system.

## TABLE 6

### Demand and Sustained Release of Corticosteroid Solution from Beads Measured by Vasoconstrictor Response

| CORTICOSTEROID* CONCENTRATION | 0.05% | | 0.25% | |
|---|---|---|---|---|
| APPLICATION | Initial Application Only | Reactivate at 6, 8, 24 Hours | Initial Application Only | Reactivate at 6, 8, 24 Hours |
| TIME (HOURS) AFTER APPLICATION | | RESPONSE PERCENT | | |
| 8 | 12.5 | 37.5 | 0 | 0 |
| 24 | 12.5 | 25 | 0 | 31.3 |
| 32 | 0 | 31.3 | 12.5 | 43.8 |

- Corticosteroid Solution in polymer delivery systems applied to both arms and excess removed.

- Application sites reactivated again on one arm at 6, 8, and 24 hours.

- Corticosteroid Solution release and effect measured by vasoconstrictor response at 8, 24, and 32 hours.

---

*Fluocinonide.

EP 0 277 211 B1

Example X

(Preparation of Beads)

A 2000 ml four-necked reaction flask equipped with a motorized stirrer, reflux condenser, thermometer, and nitrogen inlet was evacuated and purged with nitrogen. 1200 parts of deionized water, 9.6 parts of gum arabic and 9.6 parts of a sodium-based lignosulfonate (Reed lignin) available from the American Can Co. under the trademark Marasperse N-22, were charged to the reaction flask. The mixture was heated, with stirring, in an oil bath at about 50 degrees Celsius until the dispersants (gum arabic and lignosulfate) dissolved to form an aqueous phase.

To this mixture there was then added a freshly prepared solution of 90.5 parts of styrene (99.8% purity), 55 parts of commercial divinylbenzene (55.6% divinyl benzene, 42.3% ethylvinylbenzene), 2 parts of benzoyl peroxide (70% active ingredient and 30% water), and 69.4 parts of toluene (porogen). The aqueous phase and organic solution were agitated by stirring at a rate adjusted to give a plurality of droplets having an average droplet diameter of about 10-60 microns, as determined by visual observation of a sample of the droplets with an optical microscope (400X) with the droplets being stabilized by the dispersants. The reaction mixture was then heated to about 85 degrees Celsius and maintained at that temperature for about 12 hours, at the previously adjusted stirring rate, to form porous beads of cross-linked styrene/divinylbenzene copolymer having toluene entrapped within the network of pores. The mixture was then cooled and the porous polymeric beads were removed from the reaction flask by filtration. The filtered beads were washed initially three times with one liter portions of deionized water to remove the dispersants, followed by three washes with one liter portions of isopropanol to remove any residual, unreacted monomer and the toluene used as the porogen during polymerization. The beads were then dried in an oven for eight hours. The average particle diameter of these beads, which were white and opaque in appearance, indicating their macroporosity, was less than 35 microns, as measured by a mercury intrusion porosimeter or by optical microscopy.

The calculated or theoretical cross-linking, density of the purified beads is 21.01%. This density is calculated by multiplying the weight of divinylbenzene (55 parts) by the purity of the divinylbenzene (0.556) to get the actual weight of pure divinylbenzene which is then divided by the total weight of monomer (90.5 parts + 55 parts) and multiplied by 100.

The surface area of a sample of the purified beads was determined by the B.E.T. method to be 36.41 meters$^2$/gram while the pore volume was determined by nitrogen adsorption isotherm to be 0.206 ml/gram. The B.E.T. method is described in detail in Brunauer, S. Emmit, P.H., and Teller, E., J. Am. Chem. Soc., 60:309-16 (1938). The nitrogen adsorption isotherm method is described in detail in Barrett, E.P., Joyner, L.G. and Helenda, P.P., J. Am. Chem. Soc., 73:373-80 (1951).

Example XI

(Emollient)

A 30 part portion of the macroporous cross-linked polymer beads prepared as described in Example X above was mixed at room temperature with 100 ml of ethyl acetate. Then 15 parts of Carnation white Mineral Oil, U.S.A. Light, were added with stirring, and the resulting suspension was hand-stirred for a few minutes. The solvent was then allowed to evaporate to dryness. The beads contained 33% mineral oil entrapped within their macropores.

Example XII

(Emollient)

A 2000 ml four-necked reaction flask equipped with a motorized propeller-type stirrer, reflux condensor, thermometer, and nitogen inlet was evacuated and purged with nitrogen. 800 parts of deionized water, 6.4 parts of gum arabic and 6.4 parts of a sodium-based lignosulfonate (Reed lignin) available from the American Can Co. under the trademark Marasperse N-22, were charged to the reaction flask. The mixture was heated, with stirring, in an oil bath at about 60°C until the dispersants (gum arabic and lignosulfate) dissolved to form an aqueous phase.

To this mixture there was then added a freshly prepared solution of 102.3 parts of styrene (99.8% purity), 85.6 parts of commercial divinylbenzene (55.6% divinylbenzene, 42.3% ethylvinylbenzene), 5.3 parts of benzoyl peroxide (70% active ingredient and 30% water), and 190 parts of heptane (porogen). The aqueous phase and organic solution were agitated by stirring at a rate adjusted to obtain a plurality of droplets having an average droplet diameter of below about 60 microns, as determined by visual observation of a sample of the droplets with an optical microscope (400X), with the droplets being stabilized by the dispersants. This rate is approximately 1200 rpm.

The reaction mixture was then heated to about 80-90°C and maintained at that temperature for about 20 hours at the previously adjusted stirring rate, while maintaining a nitrogen flow of 1 ml/min, to form porous beads of cross-linked styrene/divinylbenzene copolymer having heptane (porogen) entrapped within the network of pores. The mixture was then cooled, diluted with 200 parts of water, and the porous polymeric beads were removed from the reaction flask by filtration. The filtered beads were washed initially 3 times with 1 liter portions of water to remove the dispersants, followed by three washes with 0.6 liter of a mixed solution of isopropyl alcohol and acetone (7:3 by weight) to remove any residual, unreacted monomer and the heptane used as the porogen during polymerization. The beads were then dried at room temperature for 20 hours and then in an oven at 100°C for 20 hours. The average particle diameter of these beads, which were white and opaque in appearance, indicating their macroporosity, was less than approximately 25 microns and they had a pore volume of 0.68 ml/g and a surface area of 58 $m^2$/g.

The calculated or theoretical cross-linking density of the purified beads is 25%. This density is calculated by multiplying the weight of divinylbenzene 85.6 parts by the purity of the divinylbenzene (0.556) to get the actual weight of monomer 102.3 parts + 85.6 parts and multiplied by 100.

The surface area of a sample of the purified beads was determined by the B.E.T. (Brunauer, Emmett and Teller) method and the pore volume was determined by mercury intrusion porosimetry.

Examples XIII and XV

(Emollient)

By repeating the procedure of Example XIII in every essential detail except for the weights of monomers used and the amount and type of porogen present, the macroporous polymer beads prepared as described in Table 7 below were obtained.

TABLE 7

| Example | Styrene | Divinylbenzene | Porogen, Parts | Calculated Cross-Linking Density, % | Average Particle Diameter, μm | Surface Area M²/g | Pore Volume ml/g |
|---|---|---|---|---|---|---|---|
| XIII | 100 | 80 | Mineral oil, 180 | 30 | 25 | 75 | 1.3 |
| XIV | 102.3 | 85.6 | Toluene, 188 | 25 | 25 | 13 | 0.004 |

**Claims**

1. A method for preparing a composition containing an active substance for providing delivery thereof, said method comprising:

(a) preparing by polymerisation of polyethylenically unsaturated monomers and monoethylenically unsaturated monomers in an immiscible phase, in the presence of a porogen, a plurality of rigid cross-linked polymer beads, wherein the beads have a degree of cross-linking density in the range from 20% to 80% (calculated as the weight of polyethylenically unsaturated monomer (or monomers) divided by the total weight of monomers); the cross-linking density being selected so that each bead defines a substantially non-collapsible internal network of pores capable of retaining the active substance but having residual porogen therein;

(b) extracting substantially all residual porogen from the pore network; and

(c) introducing the active substance in liquid form into the existing network of pores in the rigid beads so that it is held therein by capillary action.

2. A method as in claim 1, wherein the beads have an average diameter in the range from 5$\mu$m to 100$\mu$m.

3. A method as in claim 1 wherein the beads have an average diameter in the range from 10$\mu$m to 50$\mu$m.

4. A method as in claim 1, 2 or 3 wherein the rigid cross-linked polymer beads have a total pore volume in the range from 0.1 to 2.0cc/g, a surface area in the range from 1 to 500m$^2$/g, and an average pore diameter in the range from 0.001 to 3.0$\mu$m.

5. A method as in claim 4, wherein the pore volume is in the range from 0.3 to 1.0cc/g, the surface area is in the range from 20 to 200m$^2$/g, and the average pore diameter is in the range from 0.003 to 1.0$\mu$m.

6. A method as in any preceding claim, wherein the rigid polymeric beads are composed of a styrenedivinylbenzene copolymer or a methyl methacrylate-ethylene glycol dimethacrylate copolymer.

7. A method as in any preceding claim, wherein the active substance is selected from UV absorbants, insect repellant substances, steroids, and emollients.

8. A method as in any preceding claim, wherein the active substance is a UV absorbant selected from aminobenzoates, cinnamates, benzones, and salicylates; and/or an insect repellant substance selected from terpenoids, benzoquinones, aromatics, and synthetics; and/or an adrenocortical steroid.

9. A delivery system capable of retaining an active substance, said delivery system comprising a plurality of rigid cross-linked polymer beads formed by polymerisation of polyethylenically unsaturated monomers and monoethylenically unsaturated monomers in an immiscible phase in the presence of a porogen followed by extraction of substantially all residual porogen, each bead defining a rigid, substantially non-collapsible pore network which is substantially free from porogen and other retained substances; the beads being substantially free from reactive groups that would interact with an active substance; the beads being adapted to take up active substances into their existing rigid pore networks and retain them there, releasably, by capillary action.

10. A delivery system according to claim 9 adapted for use in a method according to any of claims 1 to 8.

11. A sunscreen and/or insect repellant composition comprising substantially rigid polymeric beads formed by polymerisation of polyethylenically unsaturated monomers and monoethylenically unsaturated monomers in an immiscible phase in the presence of a porogen, each bead defining a network of substantially non-collapsible polymeric beads and having a UV absorptive and/or insect repellant substance absorbed within said network of pores and retained there by capillary action, substantially all of the porogen having been extracted from the pore network prior to absorption of the substance therein; wherein said beads have a degree of cross-linking in the range from about 20% to 80% (calculated as the weight of polyethylenically unsaturated monomer (or monomers) divided by the total weight of monomers,) and an average diameter in the range from 10$\mu$m to 40$\mu$m.

12. A composition as in claim 11, wherein the pore volume is in the range from 0.3 to 1.0cc/g, the surface area is in the range from 20 to 200m$^2$/g, and the average pore diameter is in the range from 0.003 to 1.0$\mu$m.

**13.** A composition as in claim 11 or 12, wherein the UV absorptive and/or insect repellant substance comprises from 5% to 65% of the composition by weight.

**14.** A method for inhibiting exposure to ultraviolet and infrared radiation from the sun, said method comprising topical application of the sunscreen composition of claim 11,12 or 13 to skin.

**15.** A method for repelling insects, said method comprising topical application of the insect repellant composition of claim 11,12 or 13 to skin.

**Patentansprüche**

**1.** Verfahren zur Herstellung einer Zusammensetzung, die eine Wirksubstanz enthält, um für deren Abgabe zu sorgen, wobei das genannte Verfahren umfaßt:

(a) das Herstellen einer Vielzahl von starren Perlen aus vernetztem Polymer durch Polymerisation von polyäthylenisch ungesättigten Monomeren und monoäthylenisch ungesättigten Monomeren in einer unvermischbaren Phase in Gegenwart eines Porogens, wobei die Perlen einen Vernetzungsdichtegrad im Bereich von 20 bis 80 % (berechnet als das Gewicht des/der polyäthylenisch ungesättigten Monomers (oder Monomeren), dividiert durch das Gesamtgewicht an Monomeren) aufweisen und die Vernetzungsdichte so gewählt ist, daß jede Perle ein im wesentlichen nichtkollabierbares inneres Netz von Poren definiert, die fähig sind, die Wirksubstanz zurückzuhalten, aber restliches Porogen darin aufweisen;

(b) das Extrahieren im wesentlichen des gesamten restlichen Porogens aus dem Porennetz; und

(c) das Einbringen der Wirksubstanz in flüssiger Form in das bestehende Porennetz in den starren Perlen, sodaß sie durch Kapillarwirkung darin gehalten wird.

**2.** Verfahren nach Anspruch 1, worin die Perlen einen mittleren Durchmesser im Bereich von 5 $\mu$m bis 100 $\mu$m aufweisen.

**3.** Verfahren nach Anspruch 1, worin die Perlen einen mittleren Durchmesser im Bereich von 10 $\mu$m bis 50 $\mu$m aufweisen.

**4.** Verfahren nach Anspruch 1, 2 oder 3, worin die starren Perlen aus vernetztem Polymer ein Gesamtporenvolumen im Bereich von 0,1 bis 2,0 cm$^3$/g, eine Oberfläche im Bereich von 1 bis 500 m$^2$/g und einen mittleren Porendurchmesser im Bereich von 0,001 bis 3,0 $\mu$m aufweisen.

**5.** Verfahren nach Anspruch 4, worin das Porenvolumen im Bereich von 0,3 bis 1,0 cm$^3$/g liegt, die Oberfläche im Bereich von 20 bis 200 m$^2$/g liegt und der mittlere Porendurchmesser im Bereich von 0,003 bis 1,0 $\mu$m liegt.

**6.** Verfahren nach einem der vorhergehenden Ansprüche, worin die starren Polymerperlen aus einem Styrol-Divinylbenzol-Copolymer oder einem Methylmethacrylat-Äthylenglykoldimethacrylat-Copolymer bestehen.

**7.** Verfahren nach einem der vorhergehenden Ansprüche, worin die Wirksubstanz aus UV-Absorptionsmitteln, Insektenrepellents, Steroiden und Weichmachern ausgewählt ist.

**8.** Verfahren nach einem der vorhergehenden Ansprüche, worin die Wirksubstanz ein UV-Absorptionsmittel, das aus Aminobenzoaten, Cinnamaten, Benzonen und Salicylaten ausgewählt ist; und/oder ein Insektenrepellent, das aus Terpenoiden, Benzochinonen, Duftstoffen und Synthetikas ausgewählt ist; und/oder ein adrenokortikales Steroid ist.

**9.** Abgabesystem, das zum Zurückhalten einer Wirksubstanz fähig ist, wobei das genannte Abgabesystem eine Vielzahl starrer Perlen aus vernetztem Polymer umfaßt, die durch Polymerisation polyäthylenisch ungesättigter Monomere und monoäthylenisch ungesättigter Monomere in einer unvermischbaren Phase in Gegenwart eines Porogens, gefolgt von Extraktion im wesentlichen des gesamten restlichen Porogens gebildet werden, wobei jede Perle ein starres, im wesentlichen nichtkollabierbares Porennetz definiert, das im wesentlichen frei von Porogen und anderen zurückgehaltenen Substanzen ist; wobei die Perlen im wesentlichen frei von reaktionsfähigen Gruppen sind, die mit einer Wirksubstanz wechselwirken würden; wobei die Perlen dazu ausgebildet sind, durch Kapillarwirkung Wirksubstanzen in ihre bestehenden starren Porennetze aufzunehmen und sie darin freisetzbar zu halten.

10. Abgabesystem nach Anspruch 9, das zur Verwendung bei einem Verfahren nach einem der Ansprüche 1 bis 8 ausgebildet ist.

11. Sonnenschutz- und/oder Insektenrepellent-Zusammensetzung, die im wesentlichen starre Polymerperlen umfaßt, die durch Polymerisation von polyäthylenisch ungesättigten Monomeren und monoäthylenisch ungesättigten Monomeren in einer unvermischbaren Phase in Gegenwart eines Porogens gebildet sind, wobei jede Perle ein Netz aus im wesentlichen nichtkollabierbaren Poren definiert und eine UV-Strahlung absorbierende Substanz und/oder ein Insektenrepellent im genannten Porennetz aufgenommen und durch Kapillarwirkung darin gehalten aufweist, wobei im wesentlichen das gesamte Porogen aus dem Porennetz vor der Aufnahme der Substanz darin extrahiert worden ist; worin die genannten Perlen einen Vernetzungsgrad im Bereich von etwa 20% bis 80% (berechnet als das Gewicht des/der polyäthylenisch ungesättigten Monomers (oder Monomeren), dividiert durch das Gesamtgewicht an Monomeren) und einen mittleren Durchmesser im Bereich von 10 $\mu$m bis 40 $\mu$m aufweist.

12. Zusammensetzung nach Anspruch 11, worin das Porenvolumen im Bereich von 0,3 bis 1,0 cm$^3$/g liegt, die Oberfläche im Bereich von 20 bis 200 m$^2$/g liegt und der mittlere Porendurchmesser im Bereich von 0,003 bis 1,0 $\mu$m liegt.

13. Zusammensetzung nach Anspruch 11 oder 12, worin die UV-Strahlen absorbierende Substanz und/oder das Insektenrepellent 5 bis 65 Gew.-% der Zusammensetzung ausmacht.

14. Verfahren zur Hemmung des Ausgesetztseins an UV- und Infrarotstrahlung der Sonne, wobei das genannte Verfahren das topische Auftragen der Sonnenschutzzusammensetzung nach Anspruch 11, 12 oder 13 auf die Haut umfaßt.

15. Verfahren zum Abwehren bzw. Abweisen von Insekten, wobei das genannte Verfahren das topische Auftragen der Insektenrepellent-Zusammensetzung nach Anspruch 11, 12 oder 13 auf die Haut umfaßt.

**Revendications**

1. Méthode de préparation d'une composition contenant une substance active pour fournir la délivrance de celle-ci, ladite méthode comprenant :
   (a) la préparation par polymérisation de monomères insaturés polyéthyléniquement et de monomères insaturés monéthyléniquement dans une phase non miscible, en présence d'un porogène, d'une pluralité de billes de verre réticulées rigides, dans laquelle les billes ont un degré de densité de réticulation dans l'intervalle de 20% à 80% (calculé en tant que le poids du monomère (ou des monomères) insaturé polyéthyléniquement divisé par le poids total des monomères); la densité de réticulation étant choisie de sorte que chaque bille définisse un réseau interne substantiellement non extensible de pores capables de retenir la substance active mais ayant un porogène résiduel à l'intérieur;
   (b) l'extraction de substantiellement tout porogène résiduel du réseau de pores, et
   (c) l'introduction de la substance active sous forme liquide dans le réseau existant des pores dans les billes rigides de sorte qu'elle y est maintenue par action capillaire.

2. Méthode selon la revendication 1, dans laquelle les billes ont un diamètre moyen dans l'intervalle de 5$\mu$m à 100$\mu$m.

3. Méthode selon la revendication 1, dans laquelle les billes ont un diamètre moyen dans l'intervalle de 10$\mu$m à 50$\mu$m.

4. Méthode selon la revendication 1, 2 ou 3, dans laquelle les billes de polymère réticulées rigides ont un volume de pores total dans l'intervalle de 0,1 à 2,0cc/g, une surface spécifique dans l'intervalle de 1 à 500m$^2$/g, et un diamètre moyen de pores dans l'intervalle de 0,001 à 3,0$\mu$m.

**5.** Méthode selon la revendication 4, dans laquelle le volume des pores est dans l'intervalle de 0,3 à 1,0cc/g, la surface spécifique est dans l'intervalle de 20 à 200m$^2$/g, et le diamètre moyen de pore est dans l'intervalle de 0,003 à 1,0$\mu$m.

**6.** Méthode selon l'une quelconque des revendications précédentes, dans laquelle les billes polymérique rigides sont composées d'un copolymère styrène-divinylbenzène ou d'un copolymère vinyl méthacrylate-éthylène glycol diméthacrylate.

**7.** Méthode selon l'une quelconque des revendications précédentes, dans laquelle la substance active est choisie parmi les absorbants UV, les substances répulsives d'insectes, les stéroïdes, et les émollients.

**8.** Méthode selon l'une quelconque des revendications précédentes, dans laquelle la substance active est un absorbant UV choisi parmi les aminobenzoates, les cinnamates, les benzones, et les salicylates; et/ou une substance répulsive d'insecte choisie parmi les terpénoïdes, les benzoquinones, les aromatiques, et les synthétiques; et/ou un stéroïde adrénocortical.

**9.** Système de délivrance capable de retenir une substance active, ledit système de délivrance comprenant une pluralité de billes de polymère réticulées rigides formées par polymérisation de monomères insaturés polyéthyléniquement et de monomères insaturés monoéthyléniquement dans une phase non miscible en présence d'un porogène suivie par une extraction de substantiellement tout le porogène résiduel; chaque bille définissant un réseau de pores substantiellement non extensible, rigide qui est substantiellement exempt de porogène et d'autres substances retenues; les billes étant substantiellement exemptes de groupes réactifs qui interagiraient avec une substance active; les billes étant adaptées pour prendre les substances actives dans leurs réseaux de pores rigides existant et les retenant là, de façon amovible, par action capillaire.

**10.** Système de délivrance selon la revendication 9 adapté pour utilisation dans une méthode selon l'une quelconque des revendications 1 à 8.

**11.** Composition répulsive d'insecte et/ou écran solaire comprenant des billes polymériques substantiellement rigides formées par polymérisation de monomères insaturés polyéthyléniquement et de monomères insaturés monoéthyléniquement dans une phase non miscible en présence d'un porogène, chaque bille définissant un réseau de billes polymériques substantiellement non extensibles et ayant une substance répulsive d'insecte et/ou absorbant UV absorbée dans ledit réseau de pores et retenue là par action capillaire, substantiellement tout le porogène ayant été extrait du réseau de pores avant l'absorption de la substance dans celui-ci; dans laquelle lesdites billes ont un degré de réticulation dans l'intervalle d'environ 20% à 80% (calculé en tant que le poids du monomère (ou des monomères) insaturés polyéthyléniquement divisé par le poids total de monomères), et un diamètre moyen dans l'intervalle de 10$\mu$m à 40$\mu$m.

**12.** Composition selon la revendication 11, dans laquelle le volume des pores est dans l'intervalle de 0,3 à 1,0cc/g, la surface spécifique est dans l'intervalle de 20 à 200m$^2$/g, et le diamètre moyen de pores est dans l'intervalle de 0,003 à 1,0$\mu$m.

**13.** Composition selon la revendication 11 ou 12, dans laquelle la substance répulsive d'insecte et/ou absorbant UV comprend de 5% à 65% de la composition en poids.

**14.** Méthode pour inhiber l'exposition à une radiation ultraviolette et infrarouge provenant du soleil, ladite méthode comprenant l'application topique de la composition écran solaire de la revendication 11, 12 ou 13 à la peau.

**15.** Méthode pour repousser les insectes, ladite méthode comprenant l'application topique de la composition répulsive d'insecte de la revendication 11, 12 ou 13 à la peau.